(19) Europäisches Patentamt
European Patent Office
Office européen des brevets

(11) Veröffentlichungsnummer: **0 000 203**
**B1**

(12) **EUROPÄISCHE PATENTSCHRIFT**

(45) Veröffentlichungstag der Patentschrift: **01.07.81**

(21) Anmeldenummer: **78100280.3**

(22) Anmeldetag: **29.06.78**

(51) Int. Cl.³: **A 01 N 43/42, C 07 D 215/56**

(54) **1-Äthyl-7-chlor-6-fluor-4-chinolon-3-carbonsäure, deren Herstellung und Verwendung als Pflanzenbakterizid.**

(30) Priorität: **01.07.77 CH 8130/77**
**08.06.78 CH 6285/78**

(43) Veröffentlichungstag der Anmeldung:
**10.01.79 Patentblatt 79/1**

(45) Bekanntmachung des Hinweises auf die Patenterteilung:
**01.07.81 Patentblatt 81/26**

(84) Benannte Vertragsstaaten:
**BE CH DE FR GB NL**

(56) Entgegenhaltungen:
**BE - A - 863 429**
**DE - B - 2804 097**
**FR - A - 78 02 706**
**GB - A - 15 74 285**
**US - B - 344 479**

**CHEMICAL ABSTRACTS, *81*, 57742V (1974)**
**"Chemical substance index"; Seite 3753**

(73) Patentinhaber: **CIBA-GEIGY AG**
**Patentabteilung Postfach**
**CH-4002 Basel (CH)**

(72) Erfinder: **Sturm, Elmar, Dr.**
**Ziegelbündtenweg 20 B**
**CH-4147 Aesch (CH)**

(74) Vertreter: **Zumstein sen., Fritz, Dr. et al,**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 203 B1

# 1-Aethyl-7-chlor-6-fluor-4-chinolon-3-carbonsäure, deren Herstellung und Verwendung als Pflanzenbakterizid

Die vorliegende Erfindung betrifft 1 - Aethyl - 7 - chlor - 6 - fluor - 4 - chinolon - 3 - carbonsäure, bakterizide Mittel enthaltend diese Verbindung als Wirkstoff und ein Verfahren zur Bekämpfung von Erwinia-Arten durch Applikation dieser Verbindung der Formel I oder eines ihrer Salze

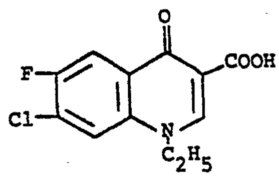

(I)

Als Salze sind solche starker organischer Basen wie Methylamin, Triäthylamin, vor allem aber solche anorganischer Alkali- und Erdalkali-Metalle sowie Ammoniak zu verstehen.

Unter den Erwinia-Arten befinden sich einige, die bedeutende, wirtschaftlich sehr nachteilige Bakteriosen hervorrufen. Es sind dies einmal die Erreger der Nassfäule an Pflanzen, z.B. E. carotovora, E. atroseptica, E. chrysanthemi, zum anderen der Erreger des Feuerbrands, E. amylovora. In der ersten, nach ihrem Haupterreger E. carotovora benannten Gruppe fasst man alle Arten der gefürchteten Nassfäulen zusammen, die nach der Ernte bei vielen Gemüse-Arten, bei Rüben und bei Kartoffeln unter ungünstigen Lagerbedingungen (hohe Luftfeuchtigkeit und Sauerstoffmangel infolge schlechter Belüftung) verheerende Schäden ausrichten können. Doch auch im Feld vermögen Erreger der E. carotovora-Gruppe das Saatgut, z.B. Kartoffelknollen, noch vor der Keimung zu zerstören ("Seed Piece decay"). Kartoffelstauden können die Symptome der Schwarzbeinigkeit ("Black leg") zeigen, Rüben, Kohl und Konnyaku können ähnlich geschädigt werden.

Zur Bekämpfung dieser Bakterien werden z.T. Tauchbehandlungen mit Na-Hypochlorit (NaOCl) oder Spritzapplikationen mit Streptomycin mit ganz ungenügendem Erfolg durchgeführt.

Demgegenüber erweist sich die halogenierte Chinoloncarbonsäure der Formel I als ausserordentlich wirksam.

Der Feuerbrand, verursacht durch das Bakterium Erwinia amylovora, ist eine der gefürchtetsten und wirtschaftlich bedeutendsten Krankheiten des Kernobstes. Dieses Bakteriose wurde erstmals an der Ostküste der USA beobachtet, durchschritt dann in einem einmaligen Seuchenzug den nordamerikanischen Kontinent und löste anfangs des zwanzigsten Jahrunderts in Kalifornien, Oregon und Washington eine verheerende Epidemie in den dortigen Apfel- und Birnen-Pflanzungen aus. Als Folge des Feuerbrandes ist heute der kommerzielle Birnenanbau in den USA hauptsächlich auf die ariden Gebiete von Kalifornien beschränkt. Im Jahr 1957 wurde der Feuerbrand in England festgestellt und etwa 10 Jahre später wurden auch auf dem europäischen Kontinent einzelne Herde dieser Krankeit beobachtet. Trotz gewaltiger Rodungsaktionen, mit denen man versuchte, diese Befallsherde auszulöschen, konnte sich der Feuerbrand ausbreiten und in Nord-Europa fest etablieren (Dänemark, Schleswig-Holstein, Holland, Regionen von Frankreich und Belgien). Vorläufig sind in den betroffenen europäischen Gebieten vor allem Büsche aus der Familie der Rosaceen (Crataegus, Cotoneaster etc.) befallen, doch am weiteren Vordringen der Krankheit auch in die grossen Obstbau-Regionen is nicht zu zweifeln.

Das stetige Vordringen dieser Krankheit beweist die Unzulänglichkeit der bisherigen Massnahmen (Streptomycin-Einsätze, Kupferpräparate u.a.)

Es wurde nun überraschend gefunden, dass die Chinoloncarbonsäure der Formel I insbesondere diese Krankheit protektiv verhüten kann.

Chinoloncarbonsäuren sind in allgemeiner Form als Therapeutika in der GB-PS. 830,832 vorgeschlagen worden. Eine pflanzenbakterizide Wirkung wird nicht erwähnt. Der überwiegende Teil der dort genannten Verbindungen ist gegen E. amylovoro völlig wirkungslos.

Weiterhin ist in Chem. Substance Index, Seite 3753, Spalte 3, Zeilen 93 bis 96 1 - Äthyl - 7 - fluor - 4 - chinolon - 3 - carbonsäure genannt.

Die anmeldungsgemäße Verbindung der Formel I besitzt, wie Vergleichsversuche zeigen, gegenüber diesen bekannten Halogen-Analogen hinsichtlich der bakteriziden Wirkung eine sprunghafte Überlegenheit.

Der Anmelder hat sich unter Bezugnahme auf die älteren Anmeldungen Nr. 863 429 in Belgien, P 28 04 097.3 in der Bundesrepublik Deutschland, 78 02706 in Frankreich und 3763/78 im Vereinigten Königreich freiwillig eingeschränkt und gesonderte Patentansprüche für die genannten Staaten vorgelegt.

Die Herstellung der Verbindung der Formel I erfolgt durch Reaktion eines Anilinderivats der Formel II mit einem Alkoxymethylenmalonsäureester unter Alkanol-Abspaltung bei 20°—160°C und Ringschluss des erhaltenen Anilinomethylenmalonsäureesters der Formel III bei erhöhter Temperatur von 200° bis 280°C und Abspaltung des alkoholischen Teils einer der Estergruppen zum 4 - Hydroxychinolin - 3 - carbonsäureester der Formel IV und nachfolgende Einführung der Aethylgruppe in 1-Position durch üliche Alkylierung mit z.B. einem Aethylhalogenid (wie Aethylbromid oder Aethyljodid). Der erhaltene Chinoloncarbonsäureester der Formel V kann mit Basen zu Verbindungen der Formel I verseift werden und liegt dabei in der Salzform vor, aus der er, falls gewünscht, durch Ansäuren in die freie Carbonsäure überführt werden kann:

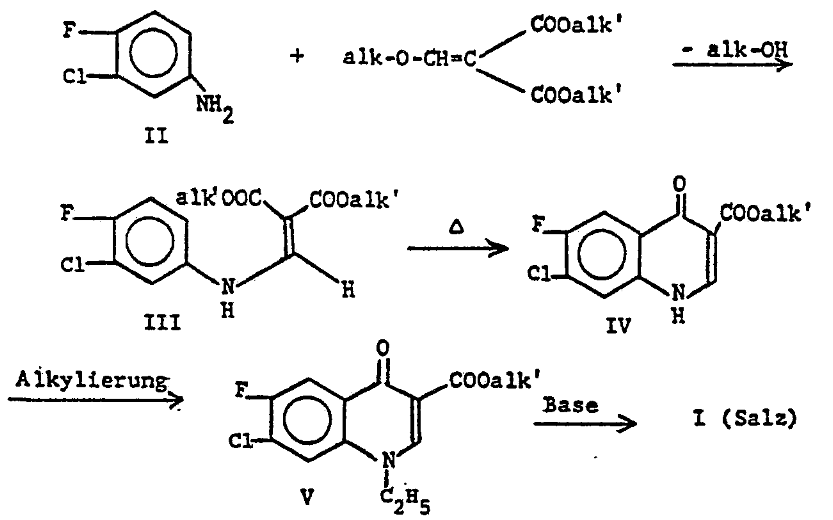


II, III, IV, V

(alk und alk' sind beliebige abspaltbare Alkylreste).

Die Chinoloncarbonsäure der Formel I ist eine stabile, in den meisten organischen Lösungsmitteln bei Normaltemperatur unlösliche Verbindung, teillöslich in Wasser, jedoch bei Zusatz von Alkalien oder starken Aminen leicht löslich. Durch Ansäuern mit z.B. Mineralsäuren lässt sie sich ausfällen bzw. in Form einer kolloidalen Lösung stabilisieren.

Die Erfindung bezieht sich auch auf die neue Verbindung der Formel I und ihre Herstellung aus Verbindungen der Formel IV.

Das folgende Herstellungsbeispiel dient der Illustration.

*Herstellung von 1 - Aethyl - 7 - chlor - 6 - fluor - 4 - chinolon - 3 - carbonsäure*

*1. Stufe*: 21,8 g 3 - Chlor - 4 - fluoranilin werden mit 32,4 g Aethoxymethylenmalonsäurediäthylester gemischt und 30 Min. auf 150° erhitzt, wobei Alkohol abdestilliert. Die Schmelze erstarrt nach dem Erkalten zu einer Kristallmasse. Man erhält so 47 g rohen 3 - Chlor - 4 - fluoranilinomethylenmalonsäurediäthylester vom Smp. 67—70°C.

*2. Stufe:* 35,5 g der voranstehenden Verbindung werden in 300 ml DOWTHERM A 1 Std. lang auf 240—250° erhitzt, wobei Alkohol abdestilliert. Nach dem Erkalten verrührt man das Gemisch mit dem doppelten Volumen Petroläther, saugt den Kristallbrei ab und erhält 20 g 7 - Chlor - 6 - fluor - 4 - hydroxychinolin - 3 - carbonsäureäthylester vom Smp. 283—290°C.

*3. Stufe*: 20 g der vorstehend beschriebenen Verbindung werden in 200 ml Dimethylformamid aufgeschlämmt und 3,9 g einer 50%igen Natriumhydrid-Dispersion zugefügt. Nach Abklingen der Wasserstoff-Entwicklung erwärmt man 1 Std. auf 80° und fügt 17 g Äthyljodid zu. Man rührt bei 80—90° Innen-Temperatur unter Rückfluss 5 Std. lang, bis eine klare Lösung entstanden ist. Das Dimethylformamid wird weitgehend im Vakuum entfernt, 200 ml Wasser und 10 g Natriumhydroxid zugesetzt und die Mischung 2 Std. unter Rückfluss gerührt. Die alkalische Lösung wird mit etwas Tierkohle versetzt, filtriert und mit konz. Chlorwasserstoffsäure sauer gestellt. Der ausgefallene Niederschlag wird abgesaugt und noch feucht aus Dimethylformamid umkristallisiert. Man erhält 15 g der Titelverbindung als farblose Kristalle, Smp. 279—281°C.

Die Verbindung der Formel I kann für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,1 bis 90%.

Zur Applikation kann die Verbindung der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel (bis zu 10%) Granulate, Umhüllungsgranulate, Imprägnierungsgranulate und Homogengranulate, Pellets (Körner) 1 bis 80%);

Flüssige Aufarbeitungsformen:

(a) in Wasser dispergierbare Wirkstoffkonzentrate: Spritzpulver (wettable powders) und Pasten (25—90% in der Handelspackung, 0,01 bis 15% in gebrauchsfertiger Lösung); Emulsions- und Lösungskonzentrate (10 bis 50%; 0,01 bis 15% in gebrauchsfertiger Lösung);
(b) Lösungen (0,1 bis 20%); Aerosole

Der Wirkstoff der Formel I vorliegender Erfindung kann beispielsweise wie folgt formuliert werden:

*Spritzpulver:* Zur Herstellung eines (a) 40%igen und (b) 10%igen Spritzpulvers werden folgende Bestandteile verwendet:

| | | | |
|---|---|---|---|
| (a) | 40 | Teile | Wirkstoff |
| | 5 | Teile | Ligninsulfonsäure-Natriumsalz, |
| | 1 | Teil | Dibutylnaphthalinsulfonsäure-Natriumsalz, |
| | 54 | Teile | Kieselsäure; |
| (b) | 10 | Teile | Wirkstoff |
| | 3 | Teile | Gemisch der Natriumsalze von gesättigten Fettalkoholsulfaten, |
| | 5 | Teile | Naphthalinsulfonsäure/Formaldehyd-Kondensat, |
| | 82 | Teile | Kaolin; |

Der Wirkstoff wird in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

*Emulgierbare Konzentrate:* Zur Herstellung eines 25%igen emulgierbaren Konzentrates werden folgende Stoffe verwendet:

| | | |
|---|---|---|
| 25 | Teile | Wirkstoff |
| 2,5 | Teile | epoxydiertes Pflanzenöl, |
| 10 | Teile | eines Alkylarylsulfonat/Fettalkoholpolyglykoläther-Gemisches |
| 5 | Teile | Dimethylformamid, |
| 57,5 | Teile | Xylol. |

Aus solchen Konzentration können durch Verdünnen mit Wasser Emulsionen der gewünschten Konzentration hergestellt werden, die besonders zur Blattapplikation geeignet sind.

Biologisches Beispiel 1

Die Prüfsubstanz, formuliert als Spritzpulver, wird in Wasser suspendiert und in verschiedenen Konzentrationen (1000 bis 100 ppm AS) auf einjährige Birnensämlinge der Sorte Bartlett bis zur Tropfnässe gesprüht. 24 Std. später erfolgt die Infektion der Bäumchen, in dem diese mit einer wässrigen Suspension eines virulenten Stammes von *Erwinia amylovora* besprüht werden. Unmittelbar nach der Infektion werden die Pflanzen wahrend 24 Std. in einem Klimaraum bei 25°C und 100% rel. Luftfeuchtigkeit aufgestellt. Anschliessend erfolgt die weitere Inkubation in einem konventionellen Gewächshaus. Sieben Tage nach der Infektion wird der Versuch ausgewertet, indem der Prozentsatz befallener Pflanzen pro Behandlung bestimmt wird. Für jede Behandlung werden 20 Bäumchen verwendet. Als Standard dient Streptomycin und als Kontrollen werden nur mit Wasser behandelte, infizierte Bäumchen herangezogen.

Im Gegensatz zu den unbehandelten Bäumchen und den mit Streptomycin behandelten tritt bei

den mit der Verbindung der Formel I behandelten Bäumen kein Befall durch E. amylovora auf.

Biologisches Beispiel 2

Kartoffelknollen werden halbiert. Die Hälften werden während 5 min. in eine wässrige Suspension mit 40 ppm bzw. 10 ppm Wirkstoff getaucht und auf Glasschalen oberflächlich betrocknen gelassen. Nach 2 Std. werden sie mit einer Bakteriensuspension von *Erwinia carotovora* besprüht. Ungetauchte Knollenhälften werden gleichfalls infiziert und dienen als Kontrolle. Anschliessend erfolgt eine Inkubation aller infizierten Knollen bei ca. 26°C und 90—100% relativer Luftfeuchtigkeit. Vier Tage nach der Infektion sind die unbehandelten Knollen breiartig zersetzt, die Wirkung an behandelten Knollen kann daher visuell bonitiert werden.

Die Verbindung der Formel I zeigt durchweg bei 40 ppm un 10 ppm volle protektive Wirkung.

**Patentansprüche für die Vertragsstaaten: CH, NL**

1. 1 - Aethyl - 6 - fluor - 7 - chlor - 4 - chinolon - 3 - carbonsäure und ihre Salze mit organischen oder anorganischen Basen.

2. Pflansenbakterizides Mittel enthaltend als mindestens eine aktive Komponente die Verbindung gemäss Anspruch 1 oder ihre Salze.

3. Verfahren zur Bekämpfung oder Verhütung von Erwinia- spp.-Bakteriosen durch Applikation der Verbindung gemäss Anspruch 1 oder eines ihrer Salze.

4. Verfahren zur Herstellung von 1 - Aethyl - 6 - fluor - 7 - chlor - 4 - chinolon - 4 - carbonsäure und ihrer Salze mit organischen oder anorganischen Basen, gekennzeichnet durch Alkylierung einer Verbindung der Formel IV

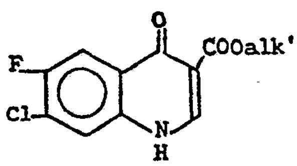

(IV)

worin alk' einen beliebigen abspaltbaren Alkylrest bedeutet, mit einem Aethylhalogenid, nachfolgende Verseifung der Estergruppe mit einer Base und, sofern gewünscht, Ueberführung des gebildeten Salzes durch Säurebehandlung in die freie Carbonsäure.

**Revendications pour les Etats contractants: CH, NL**

1. L'acide 1 - éthyl - 6 - fluoro - 7 - chloro - 4 - quinolone - 3 - carboxylique et ses sels de bases organiques ou minérales.

2. Produit bactéricide pour les végétaux, contenant au moins un composant actif consistant en le composé selon la revendication 1 ou ses sels.

3. Procédé pour traiter ou prévenir les maladies bactériennes causées par Erwinia-spp, par application du composé selon la revendication 1 ou d'un de ses sels.

4. Procédé de préparation de l'acide 1 - éthyl - 6 - fluoro - 7 - chloro - 4 - quinolone - 4 - carboxylique et de ses sels bases organiques ou minérales, caractérisé en ce que l'on alkyle un composé de formule IV

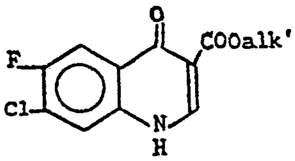

(IV)

dans laquelle alk' représente un groupe alkyle éliminable quelconque, à l'aide d'un halogénure d'éthyle, on saponifie ensuite le groupe ester par une base et, si on le désire, on convertit le sel formé en l'acide carboxylique libre par traitement à l'aide d'un acide.

**Claims for the Contracting States: CH, NL**

1. 1 - Ethyl - 6 - fluoro - 7 - chloro - 4 - quinolone - 3 - carboxylic acid and salts thereof with organic or inorganic bases.

2. A phytobactericidal composition containing, as at least one active ingredient, the compound according to Claim 1 or salts thereof.

3. A process for controlling or preventing Erwinia-spp. bacterioses by application of the compound according to Claim 1 or of one of its salts.

4. A process for producing 1 - ethyl - 6 - fluoro - 7 - chloro - 4 - quinolone - 4 - carboxylic acid and salts thereof with organic or inorganic bases, characterised by alkylation of a compound of the formula IV

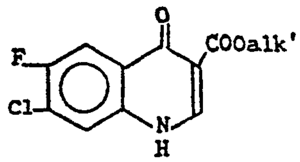

(IV)

wherein alk' is any alkyl group which can be split off, with an ethyl halide, subsequent saponification of the ester group with a base, and, if desired, conversion of the formed salt, by treatment with an acid, into the free carboxylic acid.

**Patentansprüche für die Vertragsstaaten: BE, DE, FR, GB**

1. Pflanzenbakterizides Mittel, enthaltend als mindestens eine aktive Komponente 1 - Äthyl - 6 - fluor - 7 - chlor - 4 - chinolon - 3 - carbonsäure oder ihre Salze mit organischen oder anorganischen Basen.

2. Verfahren zur Bekämpfung oder Verhütung von Erwinia-spp.-Bakteriosen durch Applikation der 1 - Äthyl - 6 - fluor - 7 - chlor - 4 - chinolon - 3 - carbonsäure oder eines ihrer Salze mit organischen oder anorganischen Basen.

3. Verfahren zur Herstellung von 1 - Aethyl - 6 - fluor - 7 - chlor - 4 - chinolon - 4 - carbonsäure und ihrer Salze mit organischen oder anorganischen Basen, gekennzeichnet durch Alkylierung einer Verbindung der Formel IV

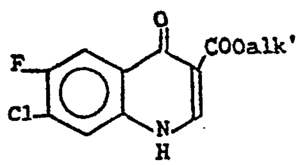

(IV)

worin alk' einen beliebigen abspaltbaren Alkylrest bedeutet, mit einem Aethylhalogenid, nachfolgende Verseifung der Estergruppe mit einer Base und, sofern gewünscht, Ueberführung des gebildeten Salzes durch Säurebehandlung in die freie Carbonsäure.

**Revendications pour les Etats contractants: BE, DE, FR, GB**

1. Produit bactéricide pour les végétaux, contenant au moins un composant actif consistant en l'acide 1 - éthyl - 6 - fluoro - 7 - chloro - 4 - quinolone - 3 - carboxylique ou ses sels de bases organiques ou minérales.

2. Procédé pour traiter ou prévenir les maladies bactériennes causées par Erwinia-spp. par application de l'acide 1 - éthyl - 6 - fluoro - 7 - chloro - 4 - quinolone - 3 - carboxylique ou d'un de ses sels de bases organiques ou minérales.

3. Procédé de préparation de l'acide 1 - éthyl - 6 - fluoro - 7 - chloro - 4 - quinoline - 4 - carboxylique et de ses sels de bases organiques ou minérales, caractérisé en ce que l'on alkyle un composé de formule IV

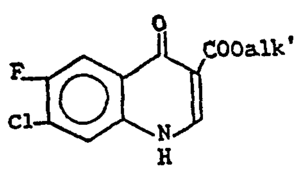

(IV)

dans laquelle alk' représente un groupe alkyle éliminable quelconque, à l'aide d'un halogènure d'éthyle, on saponifie ensuite le groupe ester par une base et, si on le désire, convertit le sel formé en l'acide carboxylique libre par traitement à l'aide d'un acide.

**Claims for the Contracting States: BE, DE, FR, GB**

1. A phytobactericidal composition containing, as at least one active ingredient, 1 - ethyl - 6 - fluoro - 7 - chloro - 4 - quinolone - 3 - carboxylic acid or a salt thereof with organic or inorganic bases.

2. A process for controlling or preventing Erwinia-spp. bacterioses by application of 1 - ethyl - 6 - fluoro - 7 - chloro - 4 - quinolone - 3 - carboxylic acid or of one of its salts with an organic or inorganic base.

3. A process for producing 1 - ethyl - 6 - fluoro - 7 - chloro - 4 - quinoline - 4 - carboxylic acid and salts thereof with organic or inorganic bases, characterised by alkylation of a compound of the formula IV

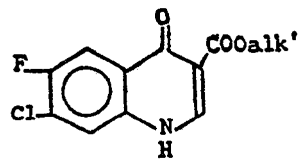

(IV)

wherein alk' is any alkyl group which can be split off, with an ethyl halide, subsequent saponification of the ester group with a base, and, if desired, conversion of the formed salt, by treatment with an acid, into the free carboxylic acid.